# EUROPEAN PATENT SPECIFICATION

(11) **EP 2 825 873 B1**
(45) Date of publication and mention of the grant of the patent: **09.03.2016**
(21) Application number: 13708835.7
(22) Date of filing: 12.03.2013
(51) Int. Cl.: G01N 27/333, G01N 33/18

(54) **DISPOSABLE SYSTEM FOR ION SELECTIVE ELECTRODES FOR LONG TERM MONITORING**
EINWEGSYSTEM FÜR IONENSELEKTIVE ELEKTRODEN FÜR LANGZEITÜBERWACHUNG
SYSTÈME JETABLE POUR ÉLECTRODES SÉLECTIVES D'IONS POUR LA SURVEILLANCE À LONG TERME

(30) Priority: 12.03.2012 US 201261609615 P
(43) Date of publication of application: 21.01.2015
(73) Proprietor: CSEM Centre Suisse d'Electronique et de Microtechnique SA - Recherche et Développement, 2007 Neuchâtel (CH)
(72) Inventor: GUENAT, Olivier, CH-3012 Berne (CH); GENERELLI, Silvia, CH-6600 Locarno (CH)
(74) Representative: GLN SA
(86) International application number: PCT/EP2013/055052
(87) International publication number: WO 2013/135733

(56) References cited:
- EP-A1- 0 579 997
- WO-A1-2009/042921
- US-A1- 2010 175 993
- RATNA TANTRA ET AL: "Integrated Potentiometric Detector for Use in Chip-Based Flow Cells", ANALYTICAL CHEMISTRY, vol. 72, no. 13, 1 July 2000 (2000-07-01), pages 2875-2878, XP055060465, ISSN: 0003-2700, DOI: 10.1021/ac000036+
- SURMEIAN M ET AL: "THREE-LAYER FLOW MEMBRANE SYSTEM ON A MICROCHIP FOR INVESTIGATION OF MOLECULAR TRANSPORT", ANALYTICAL CHEMISTRY, AMERICAN CHEMICAL SOCIETY, US, vol. 74, no. 9, 1 May 2002 (2002-05-01), pages 2014-2020, XP001132715, ISSN: 0003-2700, DOI: 10.1021/AC0112317

## Description

### Technical Field

The present invention relates to the field of ion selective electrodes used in electrochemical systems intended for long term monitoring of ionic species in an aqueous environment

### Background of the invention

In the field of water, waste water, surface and groundwater monitoring, there is a need for robust, reliable and low cost electrochemical devices, intended for the protection of water against agricultural nitrate pollution. For this purpose, ion-selective electrodes (ISE) have routinely been used for the monitoring of water, to detect nitrate, chloride, sulfate, and other ions. Most of the ion selective devices on the market are expensive and are not suited for long term monitoring. In fact, ion-selective electrodes have a limited lifetime, due to the degradation of the ion sensing membrane. This is particularly true in harsh environments, such as encountered in waste water reservoirs or in riverbeds, where further degradation of the sensor takes place through bio fouling. Electrodes that are imbedded in such harsh environments need regular maintenance. Therefore it would be desirable to replace such electrodes in a simple and convenient way. A low cost reliable assembly device minimizing the deterioration of the performance of the measurement system would by highly desirable.

Disposable solid state sensors possess significant advantages over conventional ion selective electrodes. The ability to discard the sensors after several measurement cycles but before they will ultimately fail is an interesting concept. A number of sensor designs from prior art were intended to reach this objective.

Patent CA2100557, describes a disposable ion selective electrode assembly, for use in analyzers incorporating reference elements and sensor arrays linearly positioned in parallel on a substrate. In such a configuration, all the sensors of the array are exposed simultaneously, with the consequence that their lifetime is limited.

Patent application US 2009041621 is aimed at monitoring of fluid environments (aqueous and gaseous), and in particular at devices with anti-bio fouling capabilities deployed in aquatic environments for the acquisition and monitoring of their chemical and physical conditions. In order to protect the sensors from bio fouling formation, the sensors are periodically exposed to a biocide environment after sampling sequences. This implies the need of an additional biocide reservoir, which is cumbersome for an autonomous monitoring device.

### Summary of the invention

The disadvantages of the ion selective electrode containing devices described in the foregoing prior art are substantially overcome by the disposable ion sensor system according to the present invention. This specification discloses a disposable system comprising ion selective electrodes realized by the assembly of at least two substrates, of which one substrate comprises deposited arrays of metallic contacts at one of its surfaces and which closes, after assembly, a plurality of sets of micro fluidic micro channels integrated at the surface of the other substrate, allowing to flow an electrolyte solution, an analyte solution and an ion barrier liquid to a confluent junction, where said ion barrier liquid acts as a passive valve membrane separating the electrolyte solution from the analyte solution.

The disposable system disclosed in the present patent application, comprising a plurality of ion selective electrodes formed by the assembly of the fluidic substrate and the electrode substrate, allows to be easily integrated in an analyzing device comprising at least one said system that can be signal-controllable rotated so that when one ion selective electrode of the system is being degraded, a signal is generated by the analyzing device and the degraded ion sensor will be automatically replaced by a new ion selective sensor and/or reference or pseudo-reference sensor element present in the system. This routine will be repeated until all sensors located on the system have been degraded. The system containing all the ion selective sensors can then be removed and replaced by a new system. Due to the compactness of the system, which may present the shape of a compact disc, several systems comprising each a plurality of ion selective electrodes can be easily assembled with a high density.

Other details of the invention will be found in the set of claims and are described hereafter.

### Brief description of the drawings

The nature, advantages and various additional features of the invention will appear more fully upon consideration of the illustrative embodiments in the accompanying drawings:
- Fig. 1 illustrates a top view of microfluidic microchannels integrated on a fluidic substrate;
- Fig. 2 illustrates an enlarged view of three microfluidic microchannels at their confluent junction on a fluidic substrate;
- Fig. 3 shows several profiles of cross sections of microfluidic microchannels;
- Fig. 4 shows another enlarged view of three confluent microfluid microchannels with incorporated microstructures at their confluent junction on a fluidic substrate;
- Fig. 5 illustrates a geometry of a cross section geometries of microstructures integrated at the junction between three microfluidic microchannels on a fluidic substrate;
- Fig. 6 shows a detailed view of a microfluidic microchannel structure intended to realize two ion sensitive sensors formed in junction with a single analyte microchannel;
- Fig. 7 shows a side view of a fluidic substrate and a substrate with deposited metal contacts;
- Fig. 8 shows a device allowing to rotate a system comprising a plurality of ion selective electrodes;
- Fig. 9 shows another device with different systems comprising ion selective electrodes assembled on top of each other.

### Detailed description of preferred embodiments

According to the invention, a disposable ion sensor system comprises two substrates.
- A first substrate, called fluidic substrate 6, presents several sets of microchannels integrated on its surface which are intended to draw, by capillarity, an electrolyte solution, an analyte solution and a liquid membrane at a confluent junction of said microchannels. As it will be outlined in more details further, the purpose of the liquid membrane, called hereafter also membrane, is to act as an ion barrier and valve in order to separate the analyte solution from the electrolyte solution at their confluent junction.
- A second substrate, called the electrode substrate 7, presents deposited metal contacts on one of its surfaces, opposite to the first substrate.

After assembly of the two substrates, as it will be explained hereafter, the system will contain completely formed ion selective electrodes.

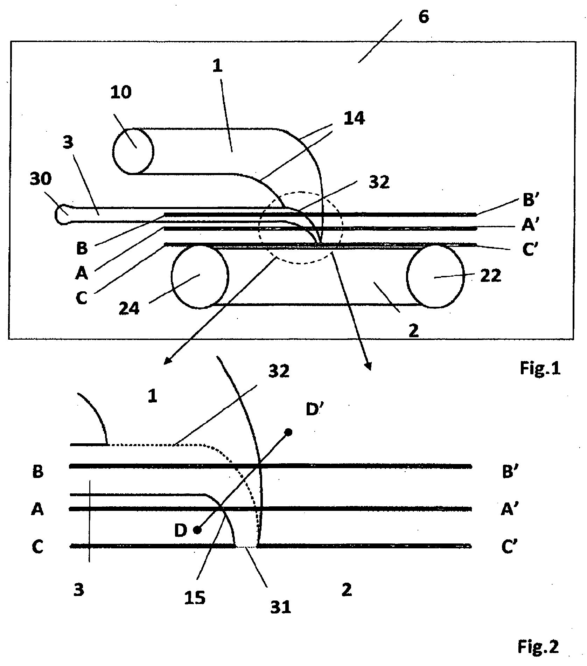

Fig.1 illustrates a configuration of surface microstructures of the fluidic substrate of the preferred configuration. A first microfluidic microchannel, called electrolyte microchannel 1, is sized to be able to draw, by capillarity, an electrolyte solution from an outside reservoir containing a known reference electrolyte solution, through an inlet 10. A second microfluidic microchannel, called the analyte channel 2, is intended to draw, by capillarity, an analyte solution from an inlet 22 to an outlet 24. A third microfluidic microchannel, called membrane microchannel 3, is intended to draw, by capillarity, a liquid membrane, and comprises an inlet 30 and is positioned in between said electrolyte microchannel 1 and said analyte microchannel 2. Fig. 2 illustrates a typical geometry of the confluent junction of the analyte microchannel 2, the electrolyte microchannel 1 and the membrane microchannel 3.

Membrane microchannel 3 and electrolyte microchannel 1 may present each a curved extremity in the region of the confluent junction. Membrane microchannel 3 is designed so that its curved extremity is connected, by an opening, to the curved extremity of the electrolyte microchannel 1. The common extremities of the electrolyte microchannel 1 and the membrane microchannel 3 form at that confluent location a single channel that is further connected to an opening at one of the sides of the analyte microchannel 1. A typical diameter of said opening is between 0.1µm to 200 µm, preferably between 1µm to 20 µm.

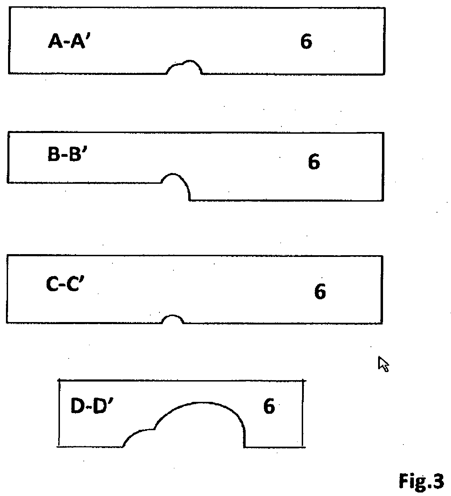

By capillarity effects, the liquid membrane can be drawn from the inlet 30 of the membrane microchannel 3 to the common confluent junction with said analyte microchannel 2 and said electrolyte microchannel 1. The liquid membrane is prevented to draw in the microchannel 1 and in the microchannel 2 by an abrupt change of the geometry of the channel as shown in figure 3 that leads to an abrupt change of the contact angle of the liquid membrane and the surface of the liquid membrane microchannel. Once the liquid membrane is held in place by surface tension effects at the common junction of said three microchannels, it will separate the electrolyte solution from the analyte solution because the electrolyte solution and analyte solution are immiscible with the liquid membrane. One of the surfaces 32 of the liquid membrane in said confluent junction will remain in contact with the electrolyte solution without fluidic exchange, while the other surface 31 of the membrane liquid in said confluent junction will remain in contact with the analyte solution without fluidic exchange.

In the system, at the contact surface 31 of the analyte solution with the liquid membrane, ions will be exchanged at said contact surface 31, producing as such an electrical potential which value is proportional to the ion content of the analyte.

The electrochemical properties and mechanism of a liquid membrane are well known to a person skilled in the art. The liquid membrane has an advantageously chosen chemical composition and property for a specific ion-selective electrode, so that the analyte solution and electrolyte solution can be separated by said liquid membrane. Its properties are based on the use of ionophores and lipophilic substances that cannot diffuse out of the membrane liquid. The ionophore in the membrane liquid allows the analyte ion to solubilize in the membrane liquid and an equilibrium of the analyte ion to be achieved at the interface of the analyte and the membrane liquid.

A great variety of compositions of membrane liquids can be used and are chosen in function of the analyte solution, the electrolyte solution, the desired surface tension properties of the deployed solutions and also the flow properties of said membrane liquid. The form of the curved part 14 of the electrolyte microchannel and the curved part 15 of the membrane microchannel are also advantageously determined so as to assure a smooth flow of the electrolyte and membrane liquid and to avoid that said fluids do not mix at their confluent junction using an advantageously chosen geometry.

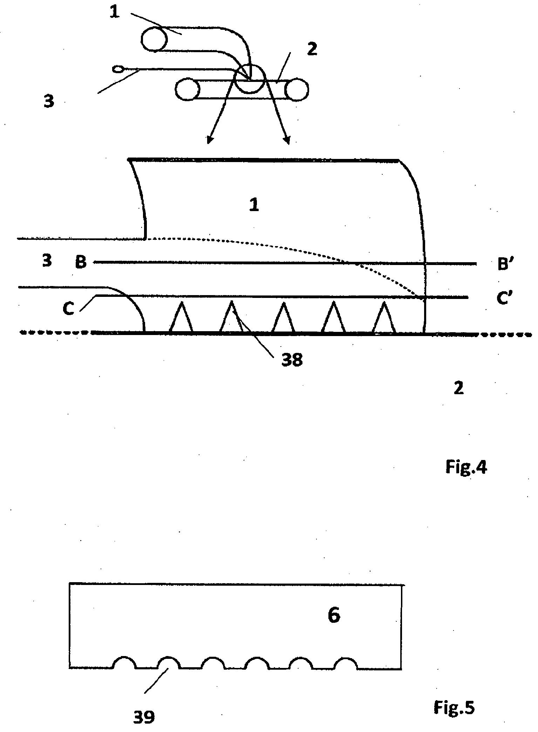

In order to explain the detailed geometry of the three dimensional structures and connection of the analyte microchannel 2, the electrolyte microchannel 1 and the membrane microchannel 3, several cross sections through the confluent junction of said three microchannels are shown in Fig. 3. In a variant design of the first embodiment, microstructures 38 can be integrated at the confluent junction of the liquid membrane and the analyte solution, as illustrated in Fig.4. The microstructures 38 are intended to improve the positional and shape stability of the membrane liquid at that area. Figure 5 illustrates a cross section 39 of such a microstructure, perpendicular to the plane of the liquid substrate. Different other geometries and positions of such an array of microstructures can be realized in the confluent region of the three fluidic micro channels. For example the microstructures can be positioned closer to the electrolyte microchannel, or several arrays of said microstructures can be organized in said confluent area. Preferably said microstructures 38 have a triangular shape in the plane of the fluidic substrate. The stabilizing microstructures can have any type of cross section perpendicular to the plane of the fluidic substrate, preferably a semi disc shaped cross section, so that the liquid membrane will stop at the interface between micro channels 3 and 2.

In order to realize complete ion selective sensors another substrate, called electrode substrate 7, is conveniently structured. It comprises deposited metallic contacts 8 on its surface, which are positioned so that they will be aligned and come in contact and close said electrolyte micro channels 1 after assembly with said fluidic substrate 6. Preferably the length of the deposited metallic contacts 8 will be chosen as long as possible and sensitively similar to the length of the electrolyte microchannel 2, this in order to maximize the stability and sensitivity of the signal to be measured. Complete ion-selective electrodes are formed at the end of the assembly process of the liquid substrate 6 with the electrode substrate 7, which surface comprising the deposited metal contacts closes completely the microfluidic microchannels and allow as such flow of fluids in their corresponding closed microchannels.

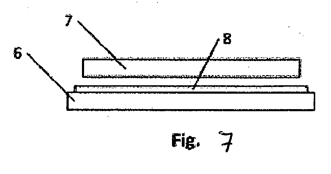

Fig. 7 illustrates a side view of said liquid substrate 6, and said electrode substrate 7.

Once the assembly of the liquid substrate 6 with electrode substrate 7 is performed, the assembled system is ready for operation and liquids can be introduced in their corresponding microchannels through their corresponding inlets.

The invention allows for different sequenced steps of the introduction of the liquids in their corresponding microchannels. In a preferred sequence of the introduction of fluids in corresponding microchannels, the membrane liquid is first introduced in the membrane microchannel 3, through its inlet 30 and drawn, by capillary effects at the confluent junction of the three said microchannels and will remain fixed at said confluent junction by surface tension effects. Thereafter the electrolyte solution and analyte solution are introduced in their respective microchannels so that each comes in contact with the stabilized membrane fluid at the confluent junction by their respective contact surfaces 31 and 32. The internal electrolyte will stop at the interface surface 32 of the membrane microchannel 3 by surface tension effects. After the introduction of the analyte solution in the analyte microchannel 2, the analyte solution will not penetrate in the membrane liquid because of the surface tension properties of the membrane liquid and measurements of specific ion contents of the analyte can start. Different variants of the liquid introduction sequence procedure can be imagined. In one of these variants, prior to the start of the analysis of the analyte, a calibration solution can be flown through the analyte microchannel and a calibration measurement is performed by a reference electrode.

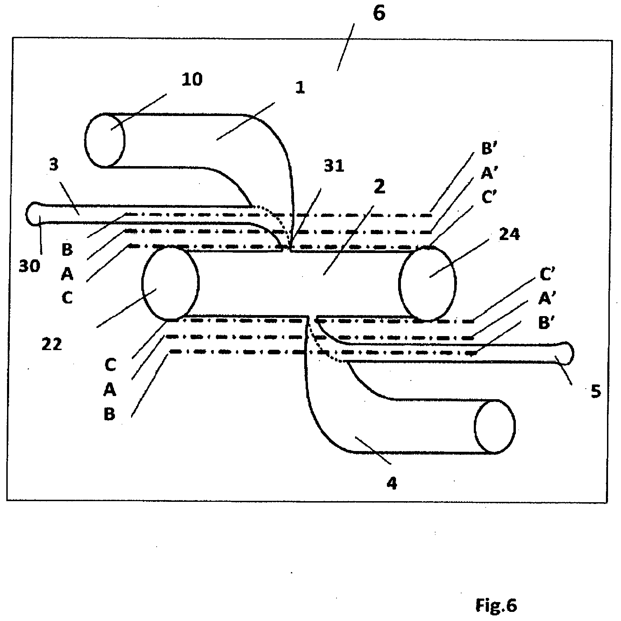

In another preferred embodiment of the invention at least one more ion selective sensor can be formed by another confluent connection of two microfluidic microchannels to the analyte microchannel 2. Fig. 6 illustrates a design allowing to realize two ion selective sensors for the same analyte microchannel 2, allowing as such the simultaneous measurement of two different ions in the analyte or to average the results of the measurement of one ion species in order to get a better accuracy. Alternatively, one of the ion-selective sensors could be used as reference channel, working as a reference or pseudo-reference electrode.

In order to achieve this configuration, a micro channel 4, intended to flow an identical or another electrolyte solution than the one introduced in microchannel 1, is integrated and separated from analyte microchannel 2 by a membrane microchannel 5. The position 33 of the confluent junction of the analyte microchannel 2 and the second membrane microchannel 5 is sensibly different than the junction location 31 of the first set of microchannels. It should be understood that the invention is not limited to the integration of 2 ion sensors for each analyte microchannel. Also, different variations of the geometries of the set of microchannels illustrated in Figs. 1 and 2 are possible.

. Ion selective sensors can be organized in a radial direction or in any other layout in said system. Also, a single system can comprise a wide variety of different possible organizations and types of ion selective sensors. In a standard system adjacent ion sensors detect different ion species. In another configuration several adjacent ion sensors detect the same ion and a next one ion sensor is designed to detect another species of ion. There is no limitation in the combination, positioning, size or numbers of the sensors in the system. It should be understood that the sets of fluidic microchannels can be oriented in different ways. The set of microchannels constituting ion selective sensors can be distributed in different ways than the circular and axial symmetric distribution.

A great number of ion selective sensors can be integrated in a system. Preferably 10 to 100 ion selective sensors can be integrated. A typical length of the electrolyte microchannel 1 is between 2 mm to 20 mm, preferably 3 mm to 15 mm. A typical depth of the microchannel 1 is 0.1 mm to 1 mm, preferably 0.2 mm to 0.5 mm. Typical width of microchannel 1 is between 0.1 mm and 1.2 mm, preferably between 0.3 mm and 1 mm.

A typical length of the analyte microchannel 2 is between 2 mm to 50 mm, preferably 5 to 30 mm. Typical depths of the micro channel1 is 0.1 mm to 1 mm, preferably between 0.3 mm and 0.6 mm. Typical width of microchannel 1 is between 0.3 mm and 2 mm, preferably between 0.5 mm and 1 mm.

A typical length of the membrane microchannel 1 is between 2mm to 20 mm, preferably 3 to 15 mm. Typical depths of the microchannel 1 is 0.05 mm to 0.3 mm, preferably between 0.1 mm and 0.2 mm. Typical width of microchannel 1 is between 0.05mm and 0.3 mm, preferably between 0.06 and 0.2 mm.

Typical diameter of the inlets and outlets are between 0.5 and 1.5 mm, preferably between 0.8 and 1.2mm. Typical mutual separation distances between the inlets 10, 20, 30 are between 4 mm to 30 mm, typically between 5 to 15 mm.

Typical width and lengths of the metal contacts are similar to the ones of the electrolyte microchannels and the thickness of the deposited metal is typically 0.1 to 2 µm, preferably 0.2 to 0.5 µm.

The fluidic substrate and the electrode substrate that form the disposable system can both be made for instance by hot embossing or by injection molding techniques or by classical precision milling. The used material of both substrates is preferably a polymeric material that can withstand rough temperature, humidity changes and solvents. COC (cyclic olefin copolymer) is such a material, other material such as COP (Cyclo Olefin Polymer), PMMA (Poly Methyl Methacrylate), PC (Polycarbonate) PEEK (Polyether Ether Ketone) or other similar polymer could be used as well. COC is well suited since it resists very well solvent such as cyclohexanone and THF, often used in ion-selective membrane solutions.

The fluidic substrate and the electrode substrate can be assembled by different well known techniques. For example they can be assembled by thermal bonding or glued using an adhesive layer, or by using a glue layer deposited on one of the substrates. The metallic contacts of the electrode substrate of the electrochemical sensors are either screen printed, evaporated or sputtered on the polymeric substrate 7 and their thickness is advantageously chosen to be very thin, preferably 1 µm, in order to allow to close the fluidic microchannels without risk of leakage.

Once the system comprising ion selective sensors is formed it can be integrated in different ways in an analyzing device.

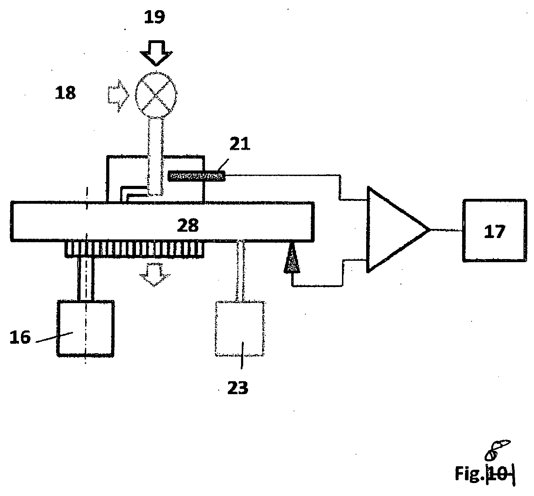

In an application of the invention the disposable system 28, which may have the size and shape of a typical compact disc having a diameter of typically 10 cm and typical thickness of 1-2 mm, is integrated in a device comprising a signal controllable motor 16 that allows to rotate the disposable system. Fig. 8 illustrates the concept of such a device. The device comprises a motor 16, preferably a step motor, a platform on which the system is fixed and advantageously chosen connectors to introduce, without leakage, liquids in their corresponding inlet and outlets of the micro channels. A container 23 is provided in order to collect the waste analyte solution. Once the measurement is performed, the signal is treated by a computer 17. If the measurement performed with the calibration solutions fails, or after a given number of sample injections, then a signal is sent to the motor, which turns the substrate, so that the next sensor faces the inlet solution.

Several variants of this aspect of the invention are possible. For instance, calibration solutions 18 can be introduced in the system via a distributor, prior to the first measurement. Then the distributor is switched and a sample measurement can be performed. The calibration 18 or the sample solution 19 flows in the disposable cartridge through a microchannel in which one or more sensors are integrated. A reference electrode 21 can either be integrated directly on the substrate or in the distributor as illustrated in Fig. 8.

It should also be pointed out that the present system and also its device could contain at least one humidity and at least one temperature sensors.

In order to protect the inlets and outlets of the ion selective sensors integrated in the system, before their use, a thin polymer layer may be deposited on one face of the system at the side of the inlets and outlets of the microfluidic microchannels. The system can be imbedded in a protective environment, preferably a liquid with anticorrosion properties. If needed, a mechanical system is provided to open and remove the thin protective polymer layer facing the inlet and outlets before the ion selective sensor has to be activated. Also, when the ion selective sensor has been positioned in its new place ready to perform measurements, a mechanical system brings a fluidic tubing comprising a fluidic connector in contact with the inlets and outlets of the set of the microfluidic microchannels of said ion selective sensor.

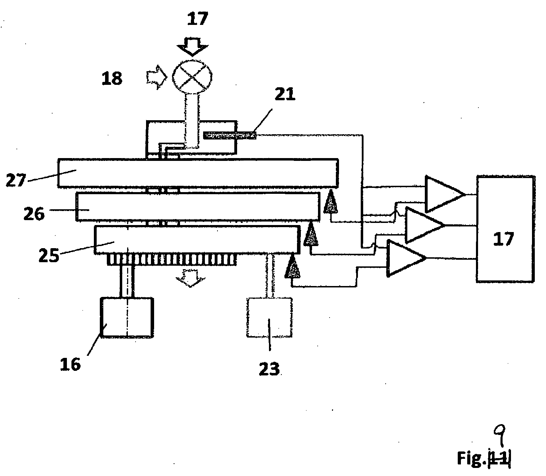

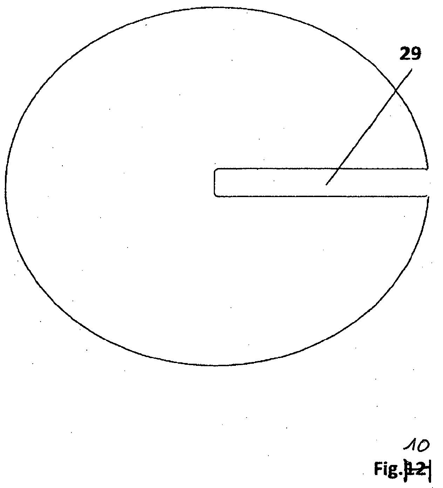

In a further embodiment of the invention, advantage is taken from the size and shape of the ion sensor system and several said systems 25, 26, 27 are assembled on top of each other in a device as shown in Fig. 9. The assembly of the different systems is designed such that each system can be rotated individually by an advantageously chosen mechanical system in which the individual rotation of the systems can be controlled by signals coming from the active ion sensors and which are treated by a computer 17 who drives the rotation of the individual systems. Different variants of the assembly of the disc shaped systems can be possible. In a preferred arrangement, illustrated in Fig. 10 the system discs comprise a radial slot 29 opened at the periphery of said system discs along a radius of the system so that the system can be removed laterally from the device. An advantageously chosen mechanical system could perform this operation automatically and assure its replacement by the introduction of a new system in the device.

## Claims

1. Disposable ion sensor system comprising an assembly comprising:
- a first substrate (6) comprising several sets of confluent microfluidic microchannels integrated at one of its surface, wherein, for each set, a first of the microfluidic microchannels (1) is designed to draw, by capillarity, an electrolyte solution, wherein, for each set, a second of said microfluidic microchannels (2) is designed to draw an analyte solution between an inlet and an outlet, and wherein, for each set, a third of the microfluidic microchannels (3) is positioned in between the first (1) and second microfluidic microchannel (2) and is designed to draw, by capillarity, a liquid membrane that has the properties of an ionic barrier fluid and passive valve intended to separate, by surface tension effects, the electrolyte solution from the analyte solution at the confluent microfluidic junction between said three microfluidic microchannels,
- a second substrate (7) comprising metallic areas (8) deposited on its surface, the second substrate (7) being assembled to the first substrate (6) so as to close the microchannels, the metallic (8) areas being superposed to the first microfluidic microchannels.

2. Disposable ion sensor system according to claim 1 wherein the length of the microfluidic microchannels are in the range of 2-20 mm, preferably 3-30mm, and their width in the range of 50-2000 µm, preferably 60-1000 µm, and their depth In the range of 50-1000 µm, preferably 100-600 µm.

3. Disposable ion sensor system according to claim 1 wherein sets of microfluidic microchannels comprise a fourth microfluidic microchannel (4) and a fifth microfluidic microchannel (5) to join said second microfluidic microchannel (2), which is intended to flow the analyte solution, and wherein said fourth microfluidic microchannel (4) is designed to draw, by capillarity, an electrolyte solution and wherein said fifth microfluidic microchannel (5) is intended to draw, by capillarity, an ionic barrier fluid that has the function of a passive valve intended to separate, by surface tension effects, the electrolyte solution from the analyte solution at the confluent microfluidic junction between said second (2), fourth (4) and fifth (5) microfluidic microchannel, wherein the fifth microfluidic microchannel (5) is positioned in between said second (2) and fourth (4) microfluidic microchannel.

4. Disposable ion sensor system according to claim 3 wherein said fourth (4) and fifth (5) microfluidic microchannel are connected to said second microfluidic microchannel (2) at a confluent junction position sensitively different than the location (31) of the confluent junction of the first (1) and third (3) microfluidic microchannel with said second microfluidic microchannel (2).

5. Disposable ion sensor system according to one of claims 1-4, wherein the substrates are made in a polymeric material.

6. Device comprising several disposable ion sensor systems according to one of the claims 1-5.

7. Device according to claim 6 wherein said ion sensor systems are assembled on top of each other, independent of each other, on a rotatable axis perpendicular to the plane of said systems connected to an signal controllable motor (16).

## Patentansprüche

1. Einweg-Ionensensorsystem, umfassend eine Einrichtung, die umfasst:
- ein erstes Substrat (6), umfassend mehrere Sätze zusammenfließender mikrofluidischer Mikrokanäle, die auf einer seiner Oberflächen integriert sind, wobei in jedem Satz ein erster der mikrofluidischen Mikrokanäle (1) derart ausgelegt ist, dass er mittels Kapillarität eine Elektrolytenlösung anzieht, wobei in jedem Satz ein zweiter der mikrofluidischen Mikrokanäle (2) derart ausgelegt ist, dass er zwischen einer Einlass- und einer Ablassöffnung eine Messlösung anzieht, und wobei in jedem Satz ein dritter der mikrofluidischen Mikrokanäle (3) zwischen dem ersten (1) und dem zweiten mikrofluidischen Mikrokanal (2) positioniert ist und derart ausgelegt ist, dass er mittels Kapillarität eine flüssige Membran anzieht, die die Eigenschaften einer ionischen Sperrflüssigkeit und eines passiven Ventils aufweist und mittels Oberflächenspannung an der zusammenfließenden mikrofluidischen Verbindungsstelle zwischen den drei mikrofluidischen Mikrokanälen die Elektrolytenlösung von der Messlösung separieren soll,
- ein zweites Substrat (7), umfassend metallische Bereiche (8), die auf seiner Oberfläche abgelagert sind, wobei das zweite Substrat (7) mit dem ersten Substrat (6) derart verbunden ist, dass es die Mikrokanäle abschließt, wobei die metallischen Bereiche (8) den ersten mikrofluidischen Mikrokanälen überlagert sind.

2. Einweg-Ionensensorsystem nach Anspruch 1, wobei die Länge der mikrofluidischen Mikrokanäle im Bereich von 2 - 20 mm, vorzugsweise 3 - 30 mm liegt, und deren Breite im Bereich von 50 - 2000 µm, vorzugsweise 60 - 1000 µm liegt, und deren Tiefe im Bereich von 50 - 1000 µm, vorzugsweise 100 - 600 µm liegt.

3. Einweg-Ionensensorsystem nach Anspruch 1, wobei Sätze mikrofluidischer Mikrokanäle einen vierten mikrofluidischen Mikrokanal (4) und einen fünften mikrofluidischen Mikrokanal (5) umfassen, um sich mit dem zweiten mikrofluidischen Mikrokanal (4), durch den die Messlösung fließen soll, zusammenzuschließen, und wobei der vierte mikrofluidische Mikrokanal (4) mittels Kapillarität eine Elektrolytenlösung anziehen soll, und wobei der fünfte mikrofluidische Mikrokanal (5) mittels Kapillarität eine ionische Sperrflüssigkeit anziehen soll, die als passives Ventil fungiert, das mittels Oberflächenspannung an der zusammenfließenden mikrofluidischen Verbindungsstelle zwischen dem zweiten (2), vierten (4) und fünften (5) mikrofluidischen Mikrokanal die Elektrolytenlösung von der Messlösung separieren soll, und wobei der fünfte mikrofluidische Mikrokanal (5) zwischen dem zweiten (2) und vierten (4) mikrofluidischen Mikrokanal positioniert ist.

4. Einweg-Ionensensorsystem nach Anspruch 3, wobei der vierte (4) und fünfte (5) mikrofluidische Mikrokanal an einer sich von der Lage (31) der zusammenfließenden Verbindungsstelle zwischen dem ersten (1) und dritten (3) mikrofluidischen Kanal erheblich unterscheidenden zusammenfließenden Verbindungsstelle mit dem zweiten mikrofluidischen Mikrokanal (2) verbunden sind.

5. Einweg-Ionensensorsystem nach einem der Ansprüche 1 - 4, wobei die Substrate aus Polymermaterial bestehen.

6. Vorrichtung, umfassend mehrere Einweg-Ionensensorsysteme nach einem der Ansprüche 1 - 5.

7. Vorrichtung nach Anspruch 6, wobei die Ionensensorsysteme unabhängig voneinander auf einer senkrecht zur Ebene der mit einem signalsteuerbaren Motor (16) verbundenen Systeme verlaufenden Achse aufeinander montiert sind.

## Revendications

1. Système capteur d' ions jetable comprenant un assemblage comprenant :
- un premier substrat (6) comprenant plusieurs ensembles de microcanaux microfluidiques confluents intégrés sur l'une de ses surfaces, dans lequel, pour chaque ensemble, un premier des microcanaux microfluidiques (1) est conçu pour soutirer, par capillarité, une solution d' électrolyte, dans lequel, pour chaque ensemble, un deuxième desdits microcanaux microfluidiques (2) est conçu pour soutirer une solution d' analyte entre une entrée et une sortie, et dans lequel, pour chaque ensemble, un troisième des microcanaux microfluidiques (3) est placé entre le premier (1) et le deuxième microcanal microfluidique (2) et est conçu pour soutirer, par capillarité, une membrane liquide qui possède les propriétés d' un fluide de barrière ionique et d' une valve passive destinée à séparer, par des effets de tension de surface, la solution d' électrolyte de la solution d' analyte au niveau de la jonction microfluidique confluente entre lesdits trois microcanaux microfluidiques,
- un second substrat (7) comprenant des zones métalliques (8) déposées sur sa surface, le second substrat (7) étant assemblé au premier substrat (6) de manière à fermer les microcanaux, les zones métalliques (8) étant superposées sur les premiers microcanaux microfluidiques.

2. Système capteur d' ions jetable selon la revendication 1 dans lequel la longueur des microcanaux microfluidiques se situe dans une plage de 2 à 20 mm, de préférence de 3 à 30 mm et leur largeur dans une plage de 50 à 2000 µm, de préférence de 60 à 1000 µm, et leur profondeur dans une plage de 50 à 1000 µm, de préférence de 100 à 600 µm.

3. Système capteur d' ions jetable selon la revendication 1 dans lequel des ensembles de microcanaux microfluidiques comprennent un quatrième microcanal microfluidique (4) et un cinquième microcanal microfluidique (5) destinés à s' adjoindre audit deuxième microcanal microfluidique (2) qui est destiné à écouler la solution d' analyte, et dans lequel ledit quatrième microcanal microfluidique (4) est conçu pour soutirer, par capillarité, une solution d' électrolyte et dans lequel ledit cinquième microcanal microfluidique (5) est destiné à soutirer, par capillarité, un fluide de barrière ionique qui a la fonction d' une valve passive destinée à séparer, par des effets de tension de surface, la solution d' électrolyte de la solution d' analyte au niveau de la jonction microfluidique confluente entre lesdits deuxième (2), quatrième (4) et cinquième (5) microcanaux microfluidiques, dans lequel le cinquième microcanal microfluidique (5) est placé entre lesdits deuxième (2) et quatrième (4) microcanaux microfluidiques.

4. Système capteur d' ions jetable selon la revendication 3 dans lequel lesdits quatrième (4) et cinquième (5) microcanaux microfluidiques sont reliés audit deuxième microcanal microfluidique (2) à une position de la jonction confluente sensiblement différente de l'emplacement (31) de la jonction confluente du premier (1) et du troisième (3) microcanal microfluidique avec ledit deuxième microcanal microfluidique (2).

5. Système capteur d' ions jetable selon l'une des revendications 1 à 4, dans lequel les substrats sont réalisés dans un matériau polymère.

6. Dispositif comprenant plusieurs systèmes capteurs d' ions jetables selon l'une des revendications 1 à 5.

7. Dispositif selon la revendication 6 dans lequel lesdits systèmes capteurs d' ions sont assemblés les uns sur les autres, indépendamment les uns des autres, sur un axe rotatif perpendiculaire au plan desdits systèmes reliés à un moteur commandé par signal (16).
